# EUROPEAN PATENT SPECIFICATION

(11) **EP 2 822 623 B1**
(45) Date of publication and mention of the grant of the patent: **01.11.2017**
(21) Application number: 13758189.8
(22) Date of filing: 22.02.2013
(51) Int. Cl.: A61M 3/02, A61M 11/06, A61M 15/08, A61M 11/00

(54) **PORTABLE NEBULIZER DEVICE**
TRAGBARE ZERSTÄUBERVORRICHTUNG
DISPOSITIF NÉBULISEUR PORTABLE

(30) Priority: 07.03.2012 US 201213414439
(43) Date of publication of application: 14.01.2015
(73) Proprietor: Medinvent, LLC, White Bear Lake, MN 55110 (US)
(72) Inventor: FLICKINGER, William J., Lino Lakes, MN 55014 (US)
(74) Representative: Parry, Simon James
(86) International application number: PCT/US2013/027267
(87) International publication number: WO 2013/133989

(56) References cited:
- DE-C- 818 247
- US-A- 2 564 400
- US-A- 2 564 400
- US-A- 4 251 033
- US-A- 5 906 198
- US-A- 5 906 198
- US-A1- 2011 040 250
- US-A1- 2011 137 290
- US-A1- 2011 137 290
- US-A1- 2011 247 610
- US-A1- 2011 247 610
- US-B1- 6 732 731
- US-B1- 6 732 731
- US-B1- 7 350 520
- US-B2- 6 619 284
- US-B2- 6 619 284
- US-B2- 6 701 916
- US-B2- 6 701 916

## Description

### TECHNICAL FIELD

The present invention generally relates to devices used for administering fluid to the upper airway in mist or droplet form, either for the irrigation of the nasal passages or the delivery of medication in a nasal nebulizer.

### BACKGROUND OF THE INVENTION

US4251033-A1 discloses a mist generator for producing mists having uniformly fine particles which includes a housing having a base, a nozzle extending upwardly therefrom and a compressed gas inlet coupled to the nozzle and a cooperating structure disposed within the housing and engaging the nozzle, the cooperating structure having a plurality of successive chambers which function with the nozzle to aspirate a liquid retained in the base to form a mist and then effect agitation of the mist, and discharge it in a downward direction within the housing and an outlet on said housing for discharging the mist.

US2011/0040250-A1 discloses a device for washing nasal cavities by a nebulized liquid, that comprises a main body, a tank for liquids including at least a nebulization chamber, and means of collection and nebulization of the treatment liquid from said nebulization chamber and delivery of the nebulized liquid towards the nasal cavities with the help of a flow of air under pressure generated by a compressor group. The main body has a cavity and the compressor group, a piloting electronic circuit and an electric battery system are all on board of a single support housed and retained in the cavity of said main body.

Devices used for administering liquid medication to a patient by way of mist or liquid droplets are generally called nebulizers and are primarily used for the delivery of medication into the lungs. These devices are best suited for the inhalation of the liquid droplets through the patient's mouth. However, some cases require the introduction of liquid droplets to the patient's nasal passages.

Current nebulizers for introduction of medication to or irrigation of the nasal passages generally comprise an air compressor, a nebulizer cup for medication, and compressor tubing to connect the compressor to the nebulizer cup. To use the nebulizer, the compressor must be placed on a sturdy surface in order to support its weight and its power supply cord must be plugged into an outlet. In general, the compressor is not a portable or lightweight device. Thus, it is the compressor tubing that provides for a convenient way of handling the nebulizer cup during use.

It would be desirable to have a nebulizer that allows for more convenient use not requiring connecting tubing, a power supply cord, or a heavy or bulky compressor. Such nebulizer should, as a whole, be lightweight and easy to handle. In addition, such a nebulizer should be small enough to be carried or moved with ease to provide for convenience while still having the power to reach the desired areas of the nasal passage. Finally, it would be desirable to have a portable nebulizer that can be readily used at any time without having to search for a nearby electrical outlet.

### SUMMARY OF THE INVENTION

The present disclosure generally provides a portable, ready-to-use device for nasal irrigation and drug delivery wherein fluid held in a canister is atomized via a contiguously attached compressed air supply to create particles sized for dispersion and retention within the nasal cavity delivered via a pressurized flow that is able to stent-open the soft tissues of the nose to deliver the resultant mist into the whole of the nasal passages without the need for the patient to create an airstream through inhalation. More specifically, the present disclosure comprises a nebulizer device that comprises a main canister section, an insert, removable cap, and a contiguous pressurized air supply directly and immediately connected to the main canister section. The nebulizer device requires no connecting tubing or power cord. Instead, the pressurized air supply forms a part of the handheld portion of the nebulizer device. Preferably, the pressurized air supply comprises an air compressor.

The main canister section comprises at least one air exit port and an air inlet, through which compressed air is introduced. The insert sits within a reservoir of the canister and comprises a fluid channel that fits over the air exit port, providing a small space between the outer surface of the air exit port and the inner surface of the fluid channel. The main canister comprises a convexly curved top portion extending down into a generally rectangular bottom opening that mates with a pressurized air supply source. When pressurized air is introduced by the handheld air compressor through the air inlet of the main canister, fluid is drawn between the air exit port and fluid channel due to a venturi effect created, thereby expelling the fluid medication as mist through an exit hole in the fluid channel.

The invention of the present disclosure provides a portable nebuliser, comprising: (a) a main canister comprising at least one air exit port; and (b) an insert with a base that fits within the canister, wherein the insert comprises at least one fluid channel that fits over the air exit port, and further wherein the fluid channel is larger in diameter than the air exit port, thereby providing a small space between the outer surface of the air exit port and the inner surface of the fluid channel; and (c) a pressurized air supply source for introducing pressurized air through the air exit port such that the fluid is expelled as a mist through an exit hole in the fluid channel, wherein the insert comprises an extension protruding outwardly to the canister, characterized in that: the air exit port extends beyond the rim of the canister; the pressurized air supply source is contiguously attached without tubing; and the fluid channel further comprises a groove extending vertically along the exterior of the fluid channel to an aperture in the extension, said aperture creating a channel to the canister.

Other optional aspects, embodiments and features of the invention will become apparent from the following detailed description when considered in conjunction with the accompanying drawings. The accompanying drawings are schematic and not intended to be drawn to scale. In the figures, each identical or substantially similar component that is illustrated in various figures is represented by a single numeral or notation. For purposes of clarity, not every component is labeled in every figure. Nor is every component of each embodiment shown where illustration is not necessary to allow those of ordinary skill in the art to understand the embodiment.

### BRIEF DESCRIPTION OF THE DRAWINGS

The novel features believed characteristic of the invention are set forth in the appended claims. The invention itself, however, as well as mode of use and advantages thereof, will best be understood by reference to the following detailed description of illustrative embodiments when read in conjunction with the accompanying drawings, wherein:
**Figure 1** is an exploded view of a nasal nebulizer in accordance with an embodiment of the present disclosure;
**Figure 2** shows a cross sectional view of a canister in accordance with an alternate embodiment of the disclosure;
**Figure 3** shows an alternate embodiment of the cover in accordance with the present disclosure;
**Figure 4** illustrates the use of the nasal nebulizer of **Figure 1** in accordance with an embodiment of the present disclosure;
**Figure 5** conceptually illustrates the function of the nasal valve in aerosol delivery that is initiated below the nasal valve;
**Figure 6** shows an embodiment of a nasal irrigator in accordance with an embodiment of the present disclosure;
**Figure 7** is a schematic cross sectional view of the assembled nasal irrigator of **Figure 6****;**
**Figure 8** shows a perspective view of an assembled nasal irrigator in accordance with an embodiment of the present disclosure;
**Figure 9a** shows an exploded view of an embodiment of a nasal nebulizer in accordance with an embodiment of the present disclosure;
**Figure 9b** shows a bottom view of an insert in accordance with an embodiment of the present disclosure;
**Figure 10** shows a perspective view of an assembled nasal nebulizer in accordance with an embodiment of the present disclosure;
**Figure 11** is a schematic cross sectional view of the assembled nasal nebulizer of **Figure 10****;**
**Figure 12a** shows an exploded view of a nasal nebulizer in accordance with an embodiment of the present disclosure;
**Figure 12b** shows a bottom view of an insert in accordance with an embodiment of the present disclosure;
**Figure 13a** shows a top perspective exploded view of the nasal nebulizer of **Figure 12****.**
**Figure 13b** shows a cross-sectional side view of an assembled nebulizer in accordance with the present disclosure;
**Figure 14** shows a perspective view of an assembled nasal nebulizer in accordance with the present disclosure;
**Figure 15** shows an exploded view of a nasal nebulizer in accordance with an embodiment of the present disclosure;
**Figure 16** shows a perspective view of an assembled nasal nebulizer in accordance with an embodiment of the present disclosure;
**Figure 17** shows a top view of the nasal nebulizer of **Figure 16****.**
**Figure 18** shows a schematic cross sectional view of a filter in accordance with an embodiment of the present disclosure;
**Figure 19A** shows an exploded view of a portable nebulizer according to an embodiment of the present invention.
**Figure 19B** shows another perspective view of the portable nebulizer shown in **Figure 19A****.**
**Figure 20** shows a front perspective view of an assembled portable nebulizer as shown in **Figures 19A** and **19B****.**
**Figure 21A** shows a perspective view of an assembled portable nebulizer according to an embodiment of the present disclosure;
**Figure 21B** shows a perspective view of a portable nebulizer as depicted in **Figure 21A****.**
**Figure 22** shows a cross sectional detailed view of a portion of the main canister of an assembled portable nebulizer according to an embodiment of the present disclosure;
**Figure 23** shows a perspective view of an assembled portable nebulizer according to an alternate embodiment of the present disclosure;

### DETAILED DESCRIPTION

The present disclosure improves upon current nebulizer designs and provides a method of delivering fluid to the nasal passages with little interaction required by the user, under sufficient pressure to stent-open the airway, and with particles of a size to ensure that the majority of the mist is retained or deposited within the upper airway. The disclosure also provides a nasal nebulizer designed to deliver a mist to the upper airway through both nostrils simultaneously. The invention is defined by the appended claim 1.

In one aspect, a nasal nebulizer of the present invention comprises a main canister with a reservoir for holding fluid, wherein the canister includes at least two air exit ports; a removable insert with a circular base that fits within said main canister, wherein the insert includes at least two fluid channels that mate with said air exit ports of the main canister, said fluid channels comprising two tubes ending in a common bell housing above the base, wherein said base holds the insert just off of the main canister surface, allowing fluid to pass between the base and main canister, and further wherein the fluid channels are larger in diameter than the air exit ports, thereby providing a small space between the outer surface of the air exit ports and the inner surface of the fluid channels that allows fluid from said reservoir to be drawn upward between the air exit ports and fluid channels and expelled as a mist in an aerosol plume through exit holes in the fluid channels due to a venturi effect created by pressurized air from the air exit ports; and at least one nozzle coupled to the bottom of said main canister to create at least one air chamber defined by the nozzle and said air exit ports, wherein the nozzle includes an air inlet for providing pressurized air into said air chamber.

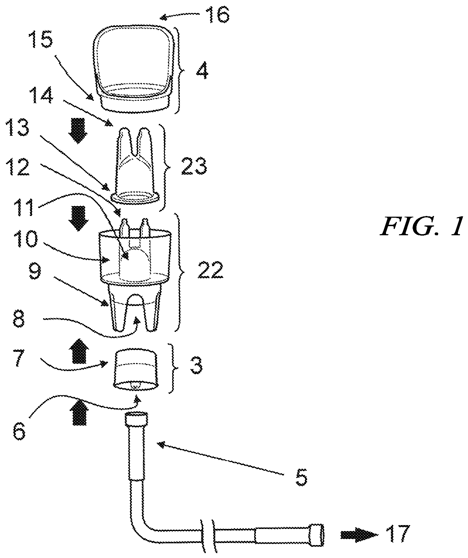

**Figure 1** is an exploded view of a nasal nebulizer in accordance with an embodiment of the present disclosure. The nasal irrigation device comprises three major sections. The first major section is the main canister **22** which has an expanded reservoir **10** that is capable of holding up to 50 ml of fluid. The inner portion of the reservoir shaped at the bottom to ensure maximal uptake of fluid to reduce waste.

The main canister **22** also includes an air chamber **11** terminating in two air exits **12** (one for each nostril) with holes sufficient to deliver an airstream that is able to atomize fluid and stent-open the upper airway. In one embodiment, each exit port **12** has at least one hole of between 0.020" and 0.060" (0.508 mm - 1.524 mm) in diameter and a web-thickness or hole length of between 0.030" and 0.200" (0.762 mm - 5.08 mm).

On the bottom of the main canister **22** is a foot section **9** that includes one or more feet for stability and an air inlet **8** for the admission of pressurized air to create the air stream through air exits **12.** The foot section **9** enables the canister **22** to stand up when set on a horizontal surface and is designed to fit into a standard docking port of an air compressor pump to enable the device to remain upright in a hands-free manner so as to remain filled with the air supply tube attached.

In the shown example, the main canister **22** has a two-step circumference to fit a holder (not shown) and provide adequate fluid volume for nasal irrigation, with the smaller diameter foot section **9** enabling the user to rest device in the holder with tube attached. In an alternate embodiment (not shown) the foot section **9** is wider than the reservoir section **10.**

The second major section of the nebulizer is the insert **23,** which is shown with a base **13** that holds the inside surface of the insert **23** just off of the outer surface of the feature within reservoir **10** of the main canister **22.** At least one channel is located in the bottom of the insert **23** to act as a conduit for fluid from the reservoir **10** to enter the base of the insert. The insert **23** includes fluid channels **14** that mate with the air exit ports **12** of the main canister **22.** Peaks or extensions may be included on the air exits **12** to ensure centering of the insert **23** and its fluid channels **14** on the air exits. Similarly, tabs may extend from the inside of the fluid channels of the insert to the outer surface of the main canister to ensure alignment. As shown, fluid channels **14** of the insert **23** comprise two tubes with one end at the bottom of the reservoir **10** and one end that is positioned in the airstream so that the airstream creates a negative pressure in each tube that draws fluid into the airstream where it is atomized (described below).

In the embodiment shown in **Figure 1****,** the atomizer outlets **12, 14** extend above the edge of the main canister **22.** However, in an alternate embodiment (not shown) the atomizer nozzles are even with or recessed within the edge or portions of the edge of the main canister.

The insert **23** is keyed in at least one location with the reservoir **10** to ensure that the insert does not rotate in relation to the exit ports **12** of the main canister and to aid in centering of the insert **23** and its fluid channels **14** on the air exits. The insert may include a feature to ensure that it is inserted into the main canister in only one orientation. In one embodiment, a loop (not shown) extends down to the saddle of the insert **23** to hold down the insert.

The fluid channels **14** are slightly larger in diameter than the air exit ports **12** of the main canister, thereby providing a small space (preferably 0.0001" to 0.010" (0.00254 - 0.254 mm)) between the outer surface of the air exit ports and the inner surface of the fluid channels. This space allows fluid from the reservoir **10** to proceed upward between the air exit ports **12** and the fluid channels **14** until being expelled by pressurized air. When the insert **23** is installed in the main canister **22,** the orifices of the fluid channels **14** are positioned relative to the air exits **12** so as to create a venturi effect with the pressurized gas expelled from the gas tubes. Because the fluid exits **14** in the insert **23** are larger than the air exits **12,** when air is forced through the air exits at an appropriate volume and speed, fluid in the reservoir **10** is drawn up into the space between the insert and air exits ports. When this fluid meets the subsequent airstream it is atomized into particles conducive to deposition in the upper airway. The airstream is sufficient to penetrate the nasal cavity above the inferior turbinate so as to deposit the fluid and provide a washing, irrigation, or deposition to the upper reaches the nasal cavity.

The exit holes of the fluid channels **14** are small enough to ensure that mist is created but large enough to ensure that the holes of the insert may be chamfered so that the walls of the exit holes are angled away from a central axis at an angle that exceeds the cone of the aerosol plume to reduce agglomeration of the mist particles upon exit, providing a more uniform particle size throughout the plume. The fluid channel size may be adjusted to change the particle size of the mist. In one embodiment the tubes have a mating section on the upper end that enables the changing of the orifice in the air stream via a series of nozzles that can be inserted into the upper end of the tubes such that the size of the nozzle orifice that is placed into the airstream is varied.

The third major section of the nebulizer is nozzle cone **3.** The nozzle **3** includes an air inlet **6** and a mating surface **7,** which attaches to the air inlet **8** of the main canister **22** to create air chamber **11** defined by the nozzle and the two exit ports **12** described above. The length of all components on the nozzle cone **3** preferably is limited so that the nozzle cone or its components do not extend past the foot section **9** on the main canister **22** when the device is assembled to enable the device to be placed on a flat surface in an upright or standing position.

Ribs may also be molded into the nozzle cone **3** to provide radial stiffness. In another embodiment, the nozzle cone is made of rigid plastic.

The mating surface between the nozzle **3** and main canister **22** is designed to ensure a tight bond can be created. In an alternate embodiment the mating surface between the nozzle **3** and main canister **22** is essentially straight.

In one embodiment, the nozzle cone **3** is attached permanently to the main canister **22.** In an alternate embodiment, the nozzle cone **3** may utilize a friction fit or have a positive connection such as a thread or other mechanism allowing the nozzle cone and main canister **22** to be disconnected for cleaning. This detachable embodiment may include an air seal such as an O-ring as well as a flange to grasp for easy disassembly.

An air supply tube **5** connects the air inlet **6** of the nozzle cone with an air supply **17.**

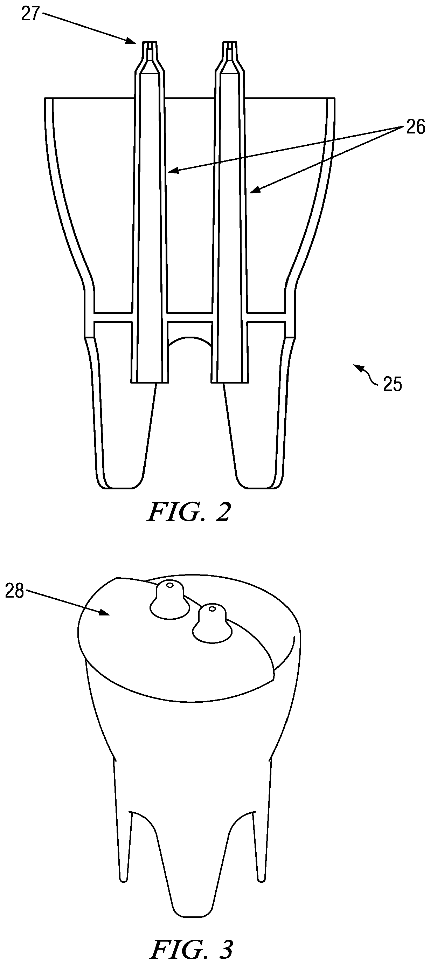

**Figure 2** shows a cross section view of a canister **25** in accordance with an alternate embodiment of the invention. In this embodiment, rather than a single air chamber and nozzle, the canister **25** includes separate air passage chambers **26** that terminate in the air exits **27.** These separate air passage chambers **26** can connect to separate air sources via separate nozzles. Alternatively, the separate air passage chambers **26** can be connected to a common air source via split tubing such as a Y or T adapter (not shown).

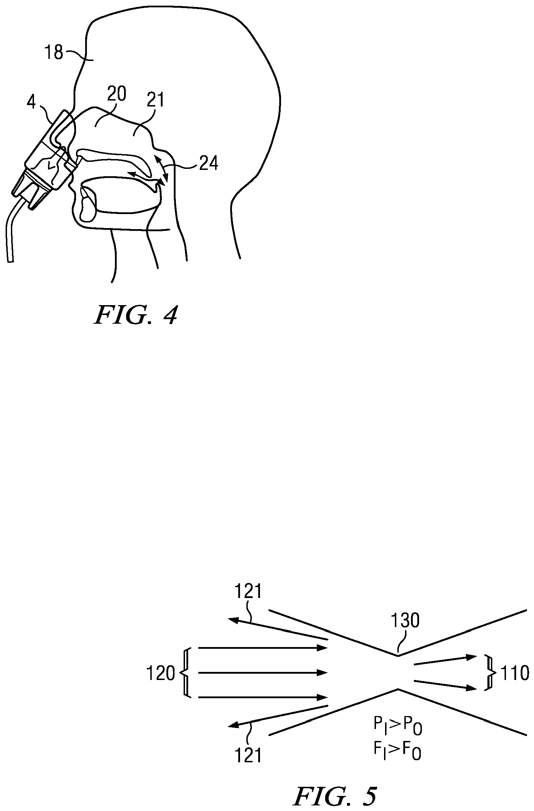

In addition to the three major sections described above, the nebulizer may include a cover **4** which has a mating surface **15** that creates an isodiametric connection to the main canister **22.** In the example shown in **Figure 1****,** the cover **4** is a broad cover region to block space between the nose, eyes and the rest of the face when in use as shown (see **Figure 4****).** In this embodiment the cover **4** is designed to confine the mist expelled from the fluid channels and shield the patient's eyes, with an opening to provide room for the patient's nose within the apparatus. The cover **4** is radiused along the distal end away from the main canister **22** to fit a broad variety of faces and is open to enable air to enter as the fluid is drawn down and capture and recycle fluid that falls off the face.

The cover may also incorporate a cross member or other device that retains the insert **23** to allow for clearance of the nose and prevent lifting of the insert at the initiation of atomization. In one embodiment a sleeve or partial sleeve extends from the cover **4** to the base of the insert **23** to hold the insert down.

**Figure 3** shows an alternate embodiment of the cover in accordance with the present invention. In this embodiment, the cover **28** is a semi-circular lid that does not block the eyes but instead retains the insert and blocks material from re-entering the main canister from the nose.

The present disclosure may incorporate a feature that guides the user to angle the spray into the nose at a set angle from 0 - 90 degrees from the plane defined as the front of the face from the chin to the forehead (i.e. the vertical plane of the face). For example, the nebulizer may include a setoff designed to set a specific angle of 30 degrees, 45 degrees, or 60 degrees from the vertical plane of the face. The setoff may be removable for various size faces or noses.

Materials suitable for construction of the nebulizer include rigid plastic, glass, metal, ceramic, carbon fiber or other rigid material, or an elastomer plastic or some combination thereof.

One embodiment of the nasal irrigation device (not shown) is egg-shaped or ovoid for better fit into the hand and a pleasing look.

**Figure 4** illustrates the use of the nasal nebulizer in accordance with the present disclosure. The nebulizer is placed over the face of the user **18** and angled such that the cover **4** blocks the eyes. The mist **20** enters the nasal passages **21,** and the patient breathes through both the mouth and nose at the same time **(24).** The mist **20** passes into the nasal passages **21** independent of the patient's breathing.

The air-fluid mixture is calibrated to achieve nasal irrigation within a short period of time, without the need for the fluid to exit the nostrils at the time of irrigation, and with a particle size that is designed to loosen the mucous or to enter the sinus cavities, as desired by the end user and not enter the pharynx or the lungs.

In one aspect, the method of nasal irrigation comprises providing fluid in a canister that includes at least two air exit ports mated to corresponding fluid channels, wherein the fluid channels are larger in diameter than the air exit ports, thereby providing a small space between the outer surface of the air exit ports and the inner surface of the fluid channels. This space allows fluid from said reservoir to be drawn upward between the air exit ports and fluid channels. Pressurized air is pumped through the air exit ports, thereby creating a venturi effect that draws fluid from said reservoir upward between the air exit ports and fluid channels and expels the fluid as a mist in an aerosol plume through exit holes in the fluid channels and into a user's nasal cavity above the inferior nasal turbinate independent of the user's breathing. The pressurized air has a pressure of 0.069 - 1.035 bar and an airflow rate of 1 - 12 liters per minute, producing a fluid delivery rate of 1 - 20 ml per minute.

The method of nasal irrigation offers a fast, convenient method of atomizing saline or medication for delivery to the nose, with a variable particle size up to 100 microns. In one embodiment, particle size is at least 10 microns.

Using an air pressure of 1 - 15psi (0.069 - 1.035bar) creates a pressurized airflow that enables the resultant air-mist stream to stent-open the soft tissues of the upper airway. Optimal performance appears to occur at 3 - 12psi (0.207 - 0.823bar), 1 - 12 lpm of airflow, and a fluid delivery rate of 1 - 20ml per minute but will vary according to the needs of the patient. Typical performance is 4 - 8psi (0.276 - 0.552bar) pressure, 3.5 - 8 lpm airflow, and 15ml per minute fluid delivery.

The resultant mist reaches the area of the nasal cavity and paranasal sinuses above the inferior nasal turbinate or chonchae to ensure that the mist reaches the areas of the sinus ostia to clear this area of the nasal cavity and enable the natural mucociliary flow to clear the sinuses.

Recent medical research has noted that the olfactory and trigeminal nerves may be used as a pathway to deliver large and small molecules to the brain and central nervous system that bypasses the blood brain barrier and first pass metabolism of intravenous and oral delivery routes. (See Dhanda, D., Frey WH 2nd, Leopold, D., Kompella, UB: "Nose-to-brain delivery approaches for drug deposition in the human olfactory epithelium." Drug Delivery Technol. 5(4), 64-72 (2005).) Frey and others have demonstrated that these nerves may be reached via the nasal mucosa overlying the olfactory cleft and cribriform plate where these nerves are concentrated. Furthermore, the frequency of dosing of many of these materials requires a delivery system that is practical and easy to use. In the case where systemic delivery of drugs via the nose is desired, maximizing the surface area of the mucosa covered by the medication may improve the amount of medication that is absorbed by the body and may reduce the variability of absorption between doses and across patients; thus improving the bioavailability of the drug and reducing the variability of bioavailability of the drug. Furthermore, by maximizing the surface area available for absorption of any given drug, the concentration required to deliver an effective dose may be reduced when compared to traditional metered dose inhaler technology, enabling more drugs to be delivered transnasally than with other systems.

However, the literature suggests that adequate delivery systems are lacking for the reliable and practical delivery of these substances to these areas. Delivery of large particles (>10 microns) of liquids in the described volumes as provided by the present invention, offers advantages over dry powder, minute volumes and high volume solutions. These advantages include covering the whole nasal mucosa, formulating drugs for patient comfort vs. concentration, reducing the inadvertent delivery of aerosolized materials to the lungs; and the ability to deliver precious materials economically and judiciously while reducing waste.

In one aspect, the present disclosure provides a method of treating neoplasms of the nasal cavity comprising fluid in a canister, wherein the canister includes a reservoir and at least two air exit ports, and wherein said fluid contains corticosteroids. The air exit ports are mated to corresponding fluid channels, wherein the fluid channels are larger in diameter than the air exit ports, thereby providing a space between the outer surface of the air exit ports and the inner surface of the fluid channels, which allows fluid from said reservoir to be drawn upward between the air exit ports and fluid channels. Pressurized air is pumped through the air exit ports, thereby creating a venturi effect that draws fluid from said reservoir upward between the air exit ports and fluid channels and expels the fluid as a mist in an aerosol plume through exit holes in the fluid channels and into a user's nasal cavity above the inferior nasal turbinate independent of the user's breathing.

The present disclosure allows for delivering steroids for the long-term control of benign neoplasms of the nasal cavity, such as inflammatory nasal polyps, granulomas, etc., without systemic doses of steroids or steroid injections. It also provides the ability to irrigate the whole nasal mucosa to manage the disruption of natural filtering and humidification often caused by ablative and reconstructive surgical treatment of neoplasms. Unlike prior art saline irrigation and nasal sprays which do not reach many of the areas of concern in the nasal vestibule and paranasal sinus areas, the nebulizer of the present invention delivers adequate moisture in less than one minute to the areas of concern. The present invention also avoids pooling of moisture that can otherwise provide a nidus for infection and cause excessive removal of the immunologic mucus blanket of the nose.

The high frequency of steroid administration needed to control neoplasm growth requires a delivery system that is practical and easy to use. The nebulizer of the present invention can deliver these steroids quickly - in less than one minute - covering the whole nasal cavity and does so without unduly exposing the body to the effects of systemic steroids.

For example, using the nebulizer of the present disclosure, 0.60 mgs of corticosteroid is typically delivered to the nasal cavity, between two and ten times the amount delivered via metered dose inhalers. In some instances, antibiotics are delivered along with the corticosteroid to treat infections such as Staphylococcus aureus. Staph aureus endotoxin has been shown to up-regulate the beta isoform of cortisol receptor (CR_{β}) in cell membranes that is responsible for inhibiting the response to corticosteroids, and it is believed that the Staph infection may contribute to steroid-resistant nasal polyps. The concurrent administration of antibiotics with the corticosteroid via the nebulizer of the present invention reduces this endotoxin effect on the cortisol receptor, thereby increasing the efficacy of the steroid therapy.

The pressure and airflow necessary to deliver material to the upper portion of the nose can be reduced if the aerosol is introduced distal of the nares at or above the nasal valve and proximal to the inferior turbinate. The present invention delivers droplets or mists with an air stream and particle sizes designed to stay in the upper airway under sufficient pressure and airflow to overcome the normal aerodynamics of the nose. Unlike prior art methods, the present invention releases mist at or above the nasal valve, thereby avoiding deflection of the fluid off the walls of the nostril and nasal valve.

Effective delivery of material to the nasal cavity requires a particle size that is large enough to fall out of the airway before reaching the oropharynx, delivered under sufficient pressure and airflow to overcome the aerodynamics of the nasal cavity. The nasal cavity is shaped to efficiently deliver air to the lungs. Air enters the nares and passes through the nasal valve, which resides approximately 1.3 cm above the nares and is the narrowest portion of the nose, with a cross-section of at approximately 0.73 cm². The nasal valve is the narrowest anatomic portion of the upper airway, resulting in the volume of air inspired nasally to be efficiently cleansed and humidified by the nasal cavity.

**Figure 5** conceptually illustrates the function of the nasal valve in aerosol delivery that is initiated below the nasal valve. Arrows **120** represent an aerosol flowing into the nasal nares. As illustrated by arrows **121,** a portion of this aerosol is reflected off the walls of the nose as the passageway narrows to the nasal valve **130.** This reflected material falls out of the nose and is either wasted or is recollected by the device to be delivered repeatedly.

The nasal valve **130** acts to reduce the flow (F) and pressure (P) of that portion of the aerosol stream that crosses the valve and enters the nasal cavity **110.** Thus, Flow in (F_{I}) is greater than Flow out (F_{O}), and Pressure in (P_{I}) is greater than Pressure out (P_{O}). As a result, aerosol entering the nasal cavity external to the nasal valve requires a higher pressure and flow rate to achieve the same aerosol distribution as an aerosol introduced internal to the nasal valve.

Air entering the nose meets additional resistance at the level of the inferior turbinate, which directs air downward along the floor of the nose along the path of least resistance. During inhalation, the airflow is dominated by the negative pressure being generated from the lower airway and is directed to the nose from the pharynx. This negative pressure and the structure of the nasal cavity conspire to direct the majority of the air through the lower third of the nose, with very little air entering the upper portion of the nose. Indeed, studies have shown that to reach the upper portion of the nose under the negative pressure of normal breathing, an aerosol must be placed very precisely at the front of the nares. To overcome the aerodynamics of the nose, the delivery system must provide a positive pressure and sufficient airflow to fill the whole nasal cavity.

Prior art devices that deliver aerosol below the nasal valve must generate higher pressure and flow rates since the valve acts to lower the pressure and flow as the aerosol passes through it. The design of the present invention is directed to the self-administration of fluid to the nasal passages of a patient while ensuring the device fits a wide variety of faces and for simplicity of design, ease of manufacturer. It requires lower pressure and airflow and produces less mess by virtue of delivery above the nasal valve, and simplicity of use, including short delivery times.

The device delivers fluid to the nasal passages with little interaction required by the user and under sufficient pressure to stent-open the airway. The invention delivers particles of a size to ensure that the majority of the mist is retained or deposited within the upper airway, while maximizing the amount of drug delivered and eliminating reflection back from the nasal valve.

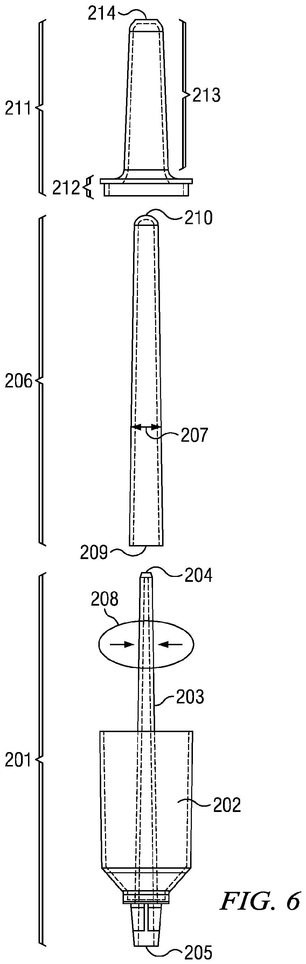

**Figure 6** shows an embodiment of a nasal irrigator in accordance with the present disclosure. The nasal irrigator comprises three main components. The first component is the main canister **201,** which has a fluid reservoir **202** and an air exit port **203** that extends above the reservoir. In one embodiment, the reservoir **202** holds up to 30 ml of fluid or medication. As shown in Figure 1, the lower portion of the reservoir is downward sloping to ensure fluid collects at the bottom, which allows maximal uptake of fluid through fluid channels (explained below), thereby minimizing waste.

The air exit port **203** has at least one exit hole **204** at the top sufficient to deliver an airstream that is able to atomize fluid and deliver the aerosol to the whole nasal cavity. In one embodiment, the exit hole **204** is between 0.020" (0.508mm) and 0.060" (1.524mm) in diameter and the air exit port has a web-thickness of between 0.030" and 0.200" (0.762 mm - 5.08 mm).

The main canister **201** also included an air inlet **205** on the bottom for the admission of pressurized air to create the air stream exiting the air exit port **203.**

In one embodiment, the main canister **201** has optional "feet" on the bottom (as shown in **Figure 1****)** for stability. The length of all components on the nozzle cone is limited so that the nozzle cone or its components do not extend past the feet on the main canister when the device is assembled to enable the device to be placed on a flat surface in an upright or standing position. The canister **201** may also be designed to fit into a standard docking port of an air compressor to enable the device to remain upright in a hands-free situation so as to be filled with the air supply tube attached.

The second main component of the nasal irrigator is an insert **206** that fits over the main canister's air exit port **203.** The insert **206** can be permanently attached to the canister **201** or it may be removable. The insert **206** has an aerosol exit **210** that is concentrically aligned with the exit hole **204** of the air outlet **203.** A peak or extension on the air exit port **203** may ensure centering of the insert over the air outlet. Similarly, tabs on the insert may be used to center the insert over the air outlet and prevent it from being moved by force. The aerosol exit **210** is slightly larger than the exit hole **204** of the air exit port **203** to enable atomization of fluid in the air stream.

The insert **206** has a tapered inner diameter **207** that is larger than and follows the contours of the outer diameter **208** of the air exit port **203.** This difference in diameter creates a space of between 0.0001" (0.00254mm) and 0.010" (0.254mm) between the inner surface of the insert **206** and the outer surface of the air exit port **203.** This space allows fluid to be drawn from the reservoir **202** through a channel **209** at the base that is sized to control the fluid flow.

The third main component of the nasal irrigator is the cover **211** that mates with the reservoir **202** of the main canister **201** and extends over the insert **206** such that the insert does not contact the nose as the device is inserted into the nasal cavity, thereby ensuring that the hole **210** in the insert **206** and the hole **204** in the air exit port **203** remain concentrically aligned. The cover **211** includes a mating surface **212** that creates a preferably isodiametric connection to the main canister **201** and extends around the nozzle formed by the insert **206** and air exit port **203.** The cover **211** extends just above the insert **206** and has its own exit hole **214** designed not to restrict the flow of the aerosol plume. In one embodiment, the cover **211** provides a cross member or other feature that secures the insert **206** to prevent lifting of the insert at the initiation of atomization.

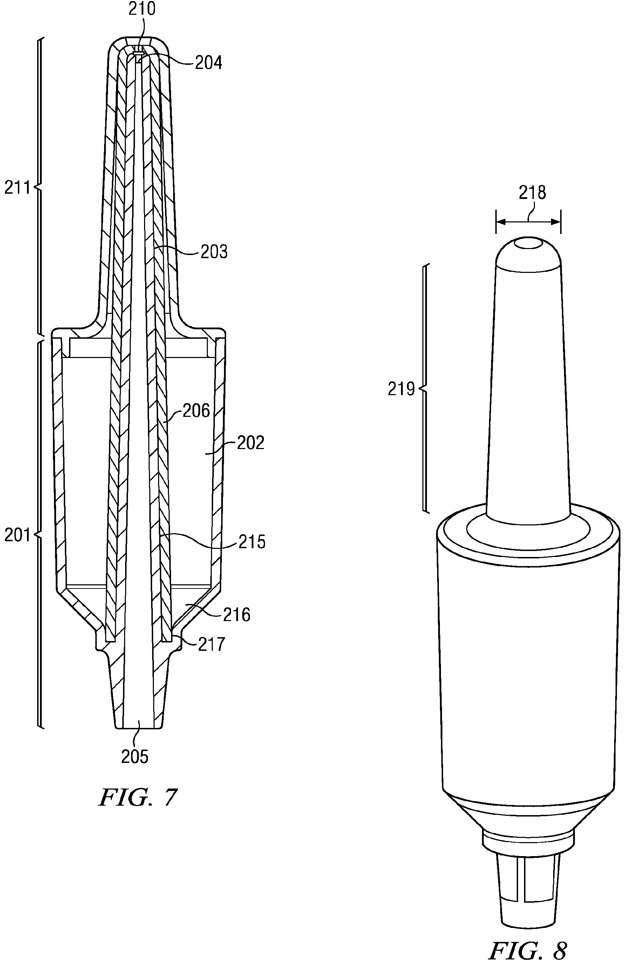

**Figure 7** is a schematic cross section view of the assembled nasal irrigator in accordance with the present invention. This view shows the alignment of the canister **201,** insert **206,** and cover **211** and the resulting fluid space **215.** When fluid is in the reservoir **202** and a pressurized air source is introduced to the system via air inlet **205,** a vacuum is created in the space **215** as air exits through outlets **204** and **210.** Because the aerosol exit hole **210** in the insert **206** is larger than the exit hole **204** of the air exit port **203,** when air is forced through the air exit port **203** at an appropriate volume and speed it creates a venturi effect as the pressurized gas is expelled, thereby drawing fluid in the reservoir **202** up into the space **215** between the insert and air outlet. When the fluid reaches the airstream between the exit holes **204, 210,** it is atomized in the airstream to create an aerosol. This aerosol is sufficiently dispersed within the nasal cavity above the inferior turbinate so as to the reach the upper nasal cavity.

The aerosol exit **210** in the insert **206** is small enough to ensure that a mist is created yet large enough to ensure that the hole can be chamfered on the outer side to reduce agglomeration of the mist particles upon exit. The aerosol exit hole **210** is chamfered so that the walls of the exit are angled away from a central axis of the hole such that the angle is greater than that of the aerosol plume. This chamfering reduces agglomeration of particles on the walls of the aerosol exit hole **210,** resulting in uniformity of particle size across the resultant aerosol plume.

The base of the insert **206** sits in a groove **217** at the base of the canister **201,** ensuring that all fluid is drawn from the bottom of the canister.

The nebulizer components of the present invention can be made from materials such as rigid plastic, glass, metal, ceramic, carbon fiber or other rigid material, an elastomer plastic, or some combination thereof.

**Figure 8** shows a perspective view of an assembled nasal irrigator in accordance with the present invention. By maintaining a sufficiently narrow nozzle assembly **218,** and a sufficiently long and smooth cover **219,** the device can be easily and atraumatically inserted into the nose of the patient so that the nozzle **218** extends to or above the nasal valve. The device is then angled by the user to obtain the best distribution based on the user's anatomy. The mist enters the nasal cavity independent of the patient's breathing.

The nasal irrigator of the present invention may also include a feature that guides the user to angle the spray into the nose to a set angle of between 0 and 90 degrees from the vertical plane of the face (defined as the front of the face from the chin to the forehead). For example, one embodiment of the nasal irrigator includes a setoff that sets a specific angle of 30 degrees from the vertical plane of the face. In another embodiment, the setoff angle is 60 degrees from vertical, and in another embodiment the setoff angle is 45 degrees from vertical. The setoff described above is removable to accommodate various size faces and noses.

The method of nasal irrigation of the present disclosure uses a variable particle size up to 100 microns under a pressure of 1-15 psi (0.069 - 1.0345 bar), creating a pressurized airflow that enables the resultant air-mist stream to reach the whole nasal cavity independent of the patient's breathing. The resultant aerosol mist reaches the area of the nasal cavity above the inferior nasal turbinate or chonchae to ensure that the mist reaches the areas of the sinus ostia to clear this area of the nasal cavity and enable the natural mucociliary flow to clear the sinuses.

By adjusting the size of the exit holes **204** and **210,** the air-fluid mixture can be calibrated to achieve nasal irrigation within a short period of time, without the need for the fluid to exit the nostrils at the time of irrigation, and with a particle size that is designed to loosen the mucous or to enter the sinus cavities, as desired by the end user. In many applications, ideally a mist of 20 microns is delivered at a rate of 0.5 ml per second.

The aerosol mist itself is typically medicated with at least one, and often two or more therapeutic agents. Possible therapeutic agents for use in the medicated mist, either alone or in combination include antibiotics, antifungal agents, corticosteroids and mucolytic agents. The mist may also be medicated with a neurologically-active agent targeting the central nervous system through the cranial nerves innervating at least a portion of the nasal cavity as well as systemically-active agents.

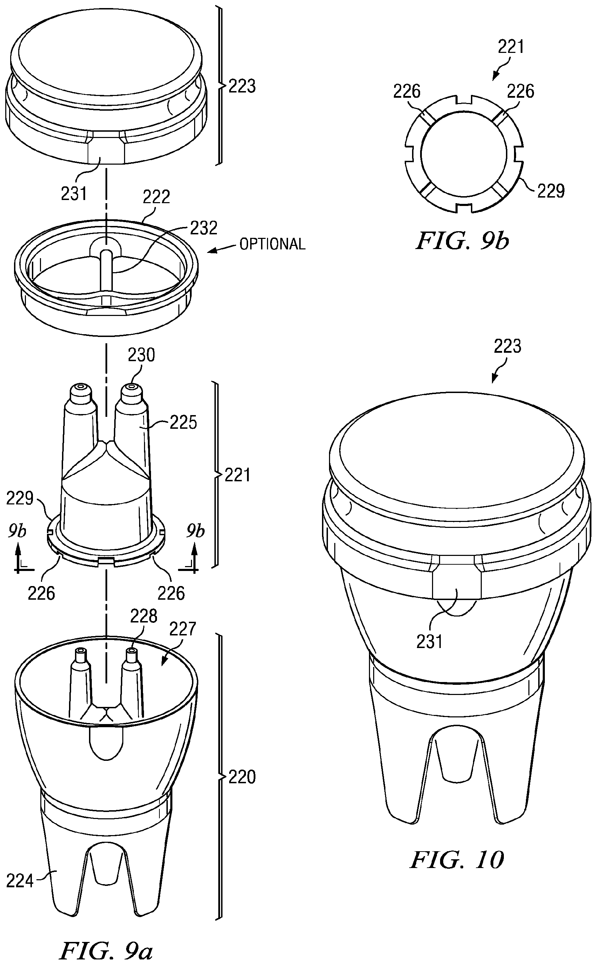

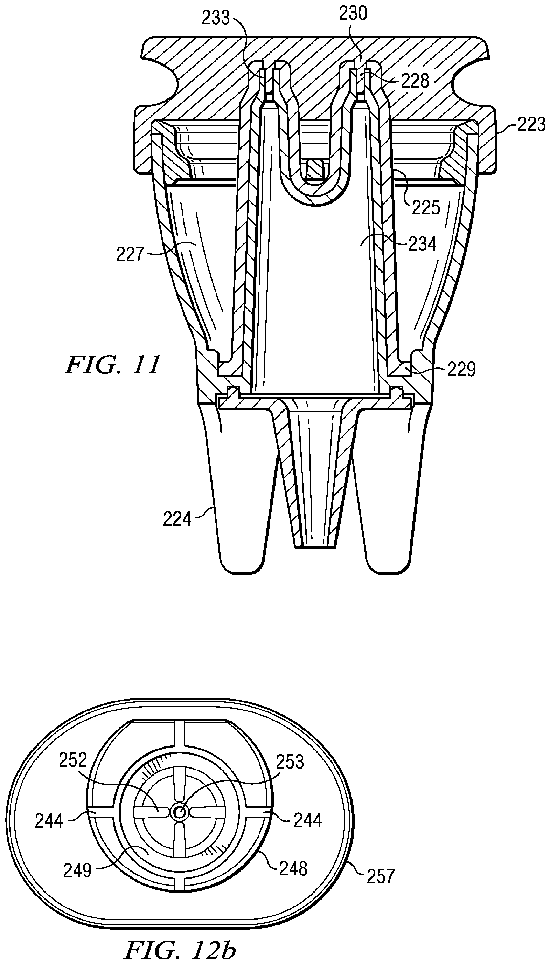

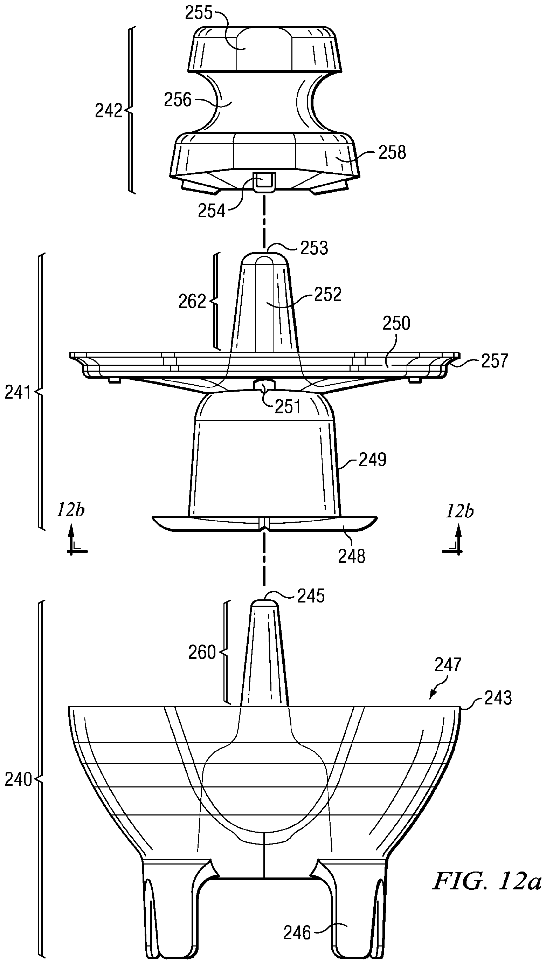

**Figure 9a** is an exploded view of an improved nasal nebulizer device according to one embodiment of the present disclosure. The device comprises a main canister **220,** an insert **221,** and a cap **223.** The main canister **220** and the insert **221** comprise many of the same characteristics of the nebulizer described with relation to **Figure 1****.** The main canister **220** comprises a rim surrounding a reservoir **227,** which can hold up to 50 mL of fluid. While the reservoir is depicted as substantially circular, it should be appreciated that the reservoir may comprise any shape. In one embodiment, the reservoir comprises an oval shape. As previously described with respect to **Figure 1****,** the main canister **220** also comprises an air chamber that terminates into at least one air exit port **228.** In one embodiment, as depicted in **Figures 9-11****,** the air chamber of the canister terminates into two air exits ports **228** (one for each nostril). In another embodiment, as best depicted in **Figures 12-14****,** the air chamber of the canister terminates into only one single air exit port.

As described above with respect to **Figure 1****,** each air exit port **228** has at least one hole of between 0.020" and 0.060" (0.508 mm - 1.524 mm) in diameter and a web-thickness or hole length of between 0.030" and 0.200" (0.762 mm - 5.08 mm). In addition, as with the embodiment of **Figure 1****,** on the bottom of the main canister **220** is a foot section **224** that includes at least one foot for stability and an air inlet (as depicted in **Figure 11****)** for the admission of pressurized air to create the air stream through air exit ports **228.** The foot section **224** enables the canister **220** to remain standing on its own when set on a substantially horizontal surface and is designed to fit into a standard docking port of an air compressor pump to enable the device to remain upright in a hands-free manner so as to remain filled with the air supply tube attached.

The insert **221** comprises a base **229** that fits within the canister **220** and sits just off the bottom of the reservoir **227.** In one embodiment, as depicted in **Figure 9****,** the base **229** is circular. However, the base may comprise any number of shapes so long as it fits within the canister. The insert **221** further comprises a fluid channel **225** that fits over the air exit port **228,** said fluid channel **225** comprising a tube portion ending in a common bell housing **234** above the base. In one embodiment, the insert comprises two fluid channels. In another embodiment, described below, the insert comprises one fluid channel.

As best depicted in **Figure 9b****,** the bottom face of the base **229** of the insert **221** comprises at least one groove **226** that forms a communication channel between the canister and the common bell housing of the insert. The groove **226** extends from the outside of the base to the inside of the insert. The base should comprise at least one groove but may also comprise more than one, as depicted in **Figure 9b****.** The number of grooves as well as the width and depth of the groove will help regulate the flow of fluid up to the point that the airflow takes over the upper limit of flow. In one embodiment, the grooves may range in width from about 0.005" to about 0.150" (0.127 mm to about 3.81 mm). In one embodiment, the grooves may range in depth from about 0.001" to about 0.050" (.0254 to about 1.27 mm). The fluid channel **225** is larger in diameter than the air exit port **228,** thereby providing a small space between the outer surface of the air exit port **228** and the inner surface of the fluid channel **225** that allows fluid from said reservoir **227** to be drawn through the communication channel and upward between the air exit port **228** and the fluid channel **225** such that the fluid is expelled as a mist in an aerosol plume through an exit hole **230** in the fluid channel due to a venturi effect created by the introduction of pressurized air from the air exit port.

In one aspect, the canister **220** and the insert **221** are preferably affixed together such that the insert **221** and the canister **220** together form an integral piece. As used herein, "affix" relates to a secure attachment between the canister and insert and may include both permanent bonding and temporary bonding, which may only be subsequently manually separated. Preferably, the affixing of the insert and canister will not interfere with or negatively affect the communication channel(s) formed by the grooves in the bottom face of the insert. In one embodiment, the insert **221** is permanently affixed or bonded to the canister **220** at the bottom face of the insert. The bond may be formed by any means known in the art including without limitation use of a solvent bond, glue UV-cured adhesives, mechanical attachment, heat forming, or radiofrequency or ultrasonic welding. In another embodiment, the canister **220** and the insert **221** may mechanically mate together, such as with a friction fit or a snap fit, to form a temporary connection between them that can be subsequently separated by the user as desired.

In yet another embodiment, where the insert comprises two fluid channels, the nasal nebulizer may further comprise a cross bar component **222** having an edge that fits around the rim of the canister. The crossbar component may comprise a single crossbar **232** that extends from one edge of the component **222** to another edge, dividing the component **222** into two substantially equal halves, as depicted in **Figure 9a** for example; or it may comprise a crossbar that extends from one edge to one or more other edges at a different locations around the circumference, dividing the enclosed space into multiple areas. In such embodiments, the crossbar component **222** may be permanently affixed or bonded to the rim of the canister **220,** thereby affixing the insert **221** to the canister **220.** The bond may be formed by any means known in the art including without limitation use of a solvent bond, glue UV-cured adhesives, mechanical attachment, heat forming, or radiofrequency or ultrasonic welding.

Covering the canister **220,** insert **221,** and optional crossbar component **222** is a cap **223** without holes therethrough. As depicted in **Figure 10****,** a cap **223** fits over the rim of the canister **220** and covers the tube portion of the insert, plugging the exit hole **230** of the fluid channel **225** and the air exit port **228** to form an airtight, hermetic seal for the nebulizer device, preventing the leakage of the fluid from the reservoir. The cap may further comprise an alignment feature or thumb hold **231** along its outer edge, which may align with a similar alignment feature or thumb hold on the exterior of the canister **220.** Thus, the nebulizer in one embodiment allows for sterile or non-sterile drug storage and serves as a carrier for the transport or shipment of medication or irrigation fluid.

**Figure 11** is a cross sectional view of an assembled nasal nebulizer comprising a canister **220,** insert **221,** optional crossbar component, and cap **223.** As best shown here in **Figure 11****,** the cap **223** may comprise sealing plugs **233** recessed within the cap, which extend through both the exit hole **230** of the fluid channel **225** and the air exit port **228.** In one embodiment, the sealing plugs **233** may be comprised of an expandable material, which will expand once removed from the top of the nebulizer device. In another embodiment, the cap may be threaded and include a gasket to form a compression seal. When ready for use, a user can remove the cap and connect an air supply to the air inlet beneath the reservoir.

A method of forming a disposable nasal nebulizer in comprises the steps of providing a canister **220** with an air exit port **228** and a rim surrounding a reservoir **227** for holding fluid; providing an insert **221** with a base **229** that fits within the canister **220,** the insert **221** comprising a fluid channel **225** that fits over the air exit port **228,** said fluid channel comprising a tube portion ending in a common bell housing **234** above the base, said base comprising at least one groove **226** along its bottom face forming a communication channel between the reservoir **227** of the canister **220** and the common bell housing **234,** wherein the fluid channel **225** is larger in diameter than the air exit port **228,** thereby providing a small space between the outer surface of the air exit port **228** and the inner surface of the fluid channel **225** that allows fluid from said reservoir **227** to be drawn through the communication channel and upward between the air exit port **228** and fluid channel **225;** and affixing the canister **220** together with the insert **221,** thereby forming one integral structure.

The providing steps (a) and (b) can comprise the step of manufacturing the canister or the insert, or both the canister and the insert. The manufacturing can be performed by any means known in the art including without limitation molding, forming, shaping or any combination thereof. The providing step (a) may also comprise the step of obtaining the canister from any manufacturer or vendor, for example. Similarly, the providing step (b) may comprise the step of obtaining the insert from any manufacturer or vendor. By way of example, in one embodiment, the insert may be permanently attached to the canister along its base **229.** Preferably, the bond would be formed such that the groove **226** remains a communication channel. Thus, the bonding should not substantially block or plug the groove **226.** In one embodiment, the insert is bonded or permanently attached along its bottom face to an interior side of the canister. A suitable solvent bond includes, for example, any plastic adhesive including without limitation ABS, acrylic, polystyrene, and polycarbonate solvents such as cyclohexanone. With the insert and canister forming one integral structure, fluid may be inserted into the reservoir **227** and the cap **223** can be placed over the rim of the canister to seal the fluid within the nebulizer device for transport or shipment.

**Figure 12a** depicts an exploded view of another embodiment of a nasal nebulizer. Similar to the above devices, the nasal nebulizer comprises a main canister **240** with an air exit port **245** and a rim **243** surrounding a reservoir **247** for holding fluid. The air exit port **245** extends beyond the rim **243** of the canister and has at least one exit hole at the top sufficient to deliver an airstream that is able to atomize fluid and deliver an aerosol. The main canister may also comprise the foot section **246** for stability. In addition, if desired, the canister may comprise one or more horizontal marks or lines to indicate specific fluid levels.

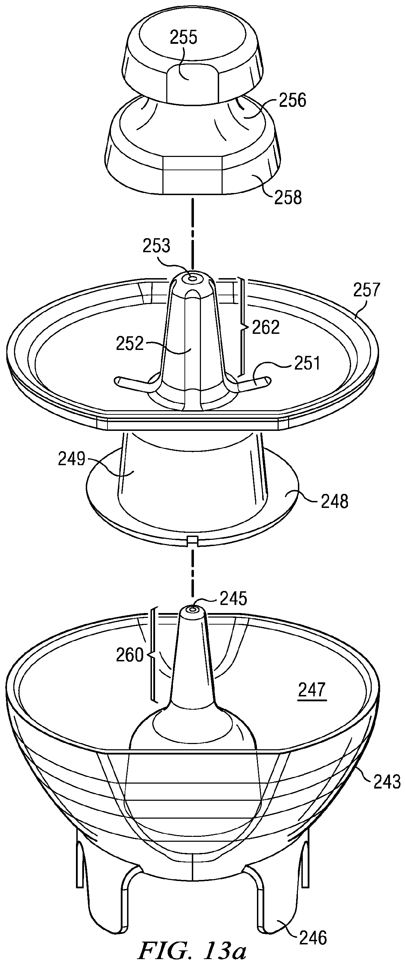

**Figure 12b** depicts an embodiment of the insert **241** with a base **248** that fits within the main canister **240,** wherein the insert has a bottom face with at least one groove **244** to form a communication channel between the canister **240** and the common bell housing **249.** In one embodiment, as depicted in **Figure 12b****,** the groove at the bottom of the insert **241** may extend from the outside edge of the bottom face to a peripheral groove surrounding the opening of the common bell housing. The insert further comprises an extension **250.** As depicted in **Figures 12a** **and** **13a****,** in one embodiment, the extension **250** protrudes outwardly from the midsection of the insert **241** above the common bell housing **249.** In other embodiments, however, the extension may also extend from another point along the insert, from the common bell housing to any point closer to the exit **253** of the fluid channel. The extension **250** forms a top, or lid, to the canister **240** that mates with the rim **243** of the canister. In one embodiment, the extension comprises a downward concave shape relative to a plane substantially perpendicular to the fluid channel; or, relative to the top surface of the lid. In one embodiment, the extension comprises a two-step diameter **257** to mate with the rim **243.** The insert **241** further comprises one or more apertures **251** around the fluid channel, each of the apertures lining up with a vertical groove **252** along the exterior of the fluid channel **262.** The groove **252** runs vertically from a point below the exit hole of the fluid channel **253** down to an aperture **251** in the extension **250.** During use, the deflected fluid will begin to flow back down the vertical groove **252.** The aperture **251** communicates with the inner chamber formed by the mating of the main canister **240** and insert **241.** As fluid exits the inner reservoir, a vacuum is created that actually pulls the deflected fluid back into the reservoir **247** through the aperture **251,** thereby ensuring maximum usage and minimized waste of the fluid.

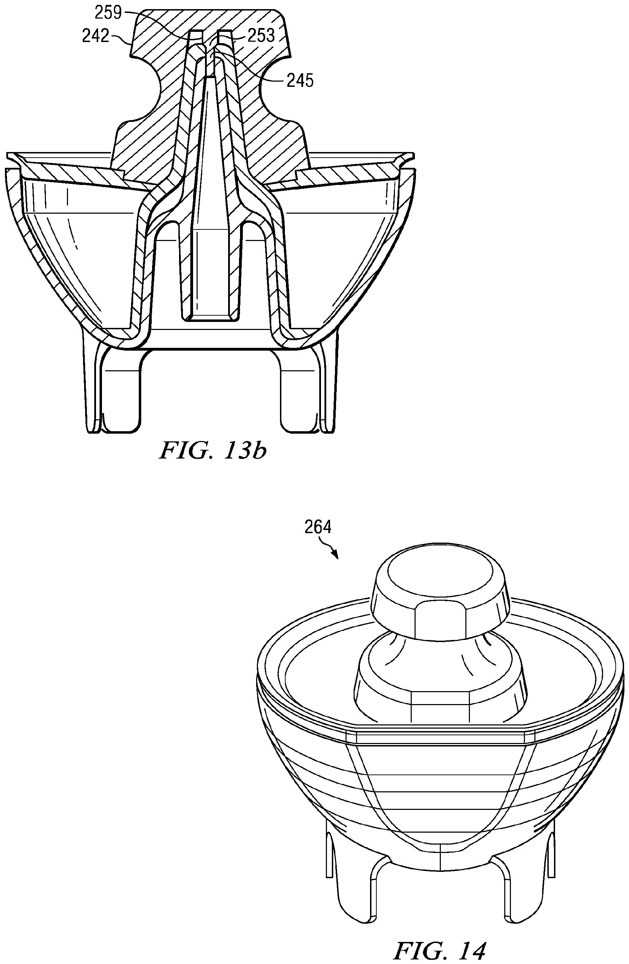

The nebulizer further comprises a cap **242** without holes that fits over and inserts into the fluid channel **253** and the air exit **port 245** to seal the reservoir from the air exit and fluid exit. The cap comprises an elongated portion **256** to ensure a good fit over the tube portion. Optionally, the cap may comprise a flattened edge **255** to help with alignment with the apertures **251** of the insert **242** and also help with the grasping the cap **242.** The bottom portion **258** of the cap mates with a portion of the top face of the extension. Thus, as best depicted in **Figure 12a****,** the bottom face of the cap **242** is relatively upwardly convex in one embodiment to mate with the downwardly concave extension **250.** The cap **242** further comprises one or more projections **254** on its bottom face, which mates with the apertures **251** of the extension. In particular, the projection **254** aligns with and seals the aperture **251** when the cap **242** is placed over the insert **241,** as best shown in **Figure 14****.** Thus, the number of projections **254** on the bottom face of the cap **242** should equal the number of apertures **251** in the insert **250.** As best depicted in **Figure 13b****,** the cap further comprises a sealing plug **259** that projects into and fits within the exit hole of the fluid channel **253** in the insert **241** and the air exit port **245,** thereby sealing the nasal nebulizer.

Similar to the embodiments described above with regard to **Figures 9-11****,** in order to make a disposable device in accordance with one aspect of the present invention, the canister **240** and the insert **241** are affixed together such that the insert **241** and the canister **240** together form an integral or single piece. In embodiments comprising an extension **250** extending from the insert to the rim of the canister (as depicted in **Figures 12-13****),** the extension may form a top that mates with the rim of the canister and the edges of the extension may be permanently affixed to the rim of the canister. Thus, in one embodiment, it is the extension that is permanently affixed to the rim of the canister by way of bonding, for example. In another embodiment, the extension may form a top that mates together with a portion of the canister. A suitable solvent bond includes, for example, any plastic adhesive including without limitation ABS, acrylic, polyacetal, polyethylene, polyester, polypropylene, polystyrene, or polycarbonate solvent, UV-cured adhesive, heat or ultrasonic welding or over molding of materials. Bonding with such materials can be performed by any means known in the art. Having the insert and canister as a single integral piece, fluid may be inserted into the reservoir **247** and the cap **242** can be placed over the exit hole **253** and the aperture(s) **251** of the insert **241** to seal the fluid within the nebulizer device for transport or shipment. The cap sits over the tube portion of the fluid channel and the fluid within the reservoir remains sealed within the nebulizer device until ready for use. **Figure 14** depicts an assembled, sealed device **260** ready for transport.

As with the above embodiments, the orifices of the fluid channels should be positioned relative to the air exits so as to create a venturi effect with the pressurized gas expelled from the gas tubes. Thus, the affixing step should account for this positioning. Because the fluid channel exits in the insert are larger than the air exits, when air is forced through the air exits at an appropriate volume and speed, fluid in the reservoir is drawn up into the space between the insert and air exits ports. When this fluid meets the subsequent airstream it is atomized into particles conducive to deposition in the upper airway.

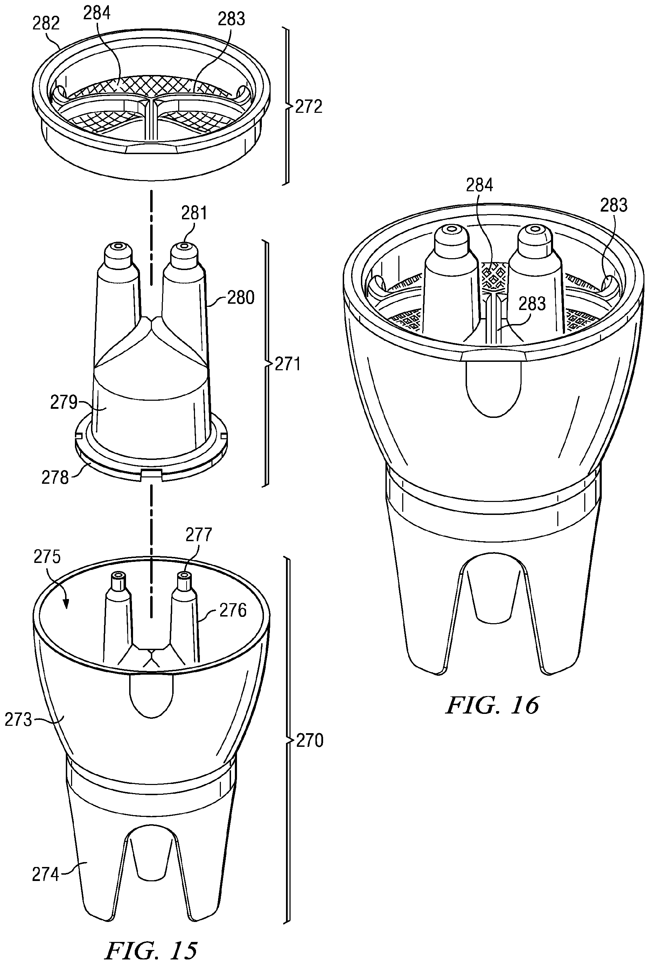

**Figure 15** is an exploded view of an embodiment of a nasal nebulizer device comprising a canister section **270,** an insert **271,** and a filter **272.** Similar to the above devices, the canister section **270** comprises a canister **273** with reservoir **275** and an air exit port **276** having an exit hole **277.** The canister section **270** also comprises one or more feet **274** beneath the canister **273;** and the insert **271** comprises a base **278** that fits within the reservoir of the canister and at least one fluid channel **280** with an exit hole **281.** As described above, the insert and canister section once formed, shaped, molded or obtained, are affixed to one another.

In one embodiment, the nasal nebulizer device further comprises a filter component **272** that may be inserted over the insert **271.** The filter component **272** comprises a filter **284** comprised of a mesh structure with holes small enough to prevent any particulate matter or mucus that runs out of the nose from entering the reservoir **275,** while allowing the irrigating or medicating fluid to run back into the reservoir **275** to be re-circulated or re-used. Suitable materials from which to create the filter are plastic, metal, carbon fiber, or other fiber. In embodiments comprising more than one fluid channel, the filter component also comprises a crossbar component **283.** In one embodiment, the crossbar **283** is an integral part of the filter component **272.** However, it should be understood that the crossbar **283** could also form a separate component, which is detached from the filter, and remains optional.

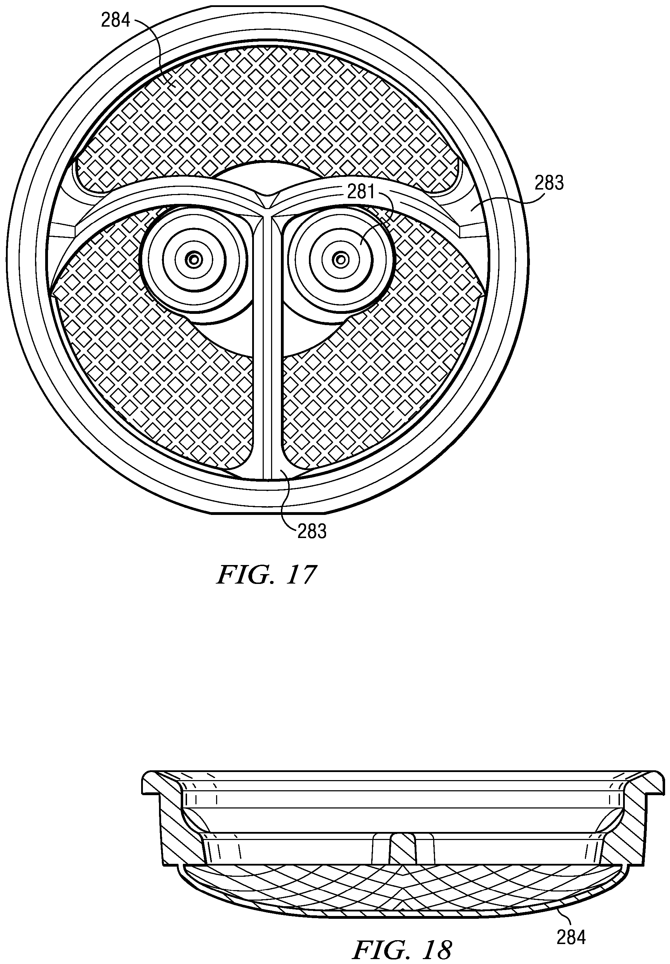

**Figure 16** is a perspective view of an assembled nebulizer having an insert having two fluid channels **280** and a filter **284** with the optional crossbar **283,** wherein the insert is affixed to the canister to form one single integral structure. As described above, in one embodiment, the insert is affixed to the canister by way of bonding. The bonding may comprise the joining of the bottom face of the insert base to the canister or the joining of the periphery of the base to the canister. In one embodiment, the insert may be affixed to the canister by permanently bonding the periphery **282** of the filter to the rim of the insert. As best depicted in **Figure 17****,** the filter **284** surrounds the tube portion **280** of the insert and extends from the rim of the canister to the tube portion **280,** substantially covering the opening of the canister such that when in use, the filter prevents particulate matter from entering the reservoir.

With reference to **Figures 12** **and** **13****,** where the nasal nebulizer comprises an extension, in one embodiment, a filter entirely covers or fits within the apertures **251** in the extension **250** to similarly keep particular matter out of the reservoir and separate from the fluid for re-circulation. The filter may slide over the fluid channel **of 241** or may be bonded over or under the apertures **251** or even molded into the insert **241.**

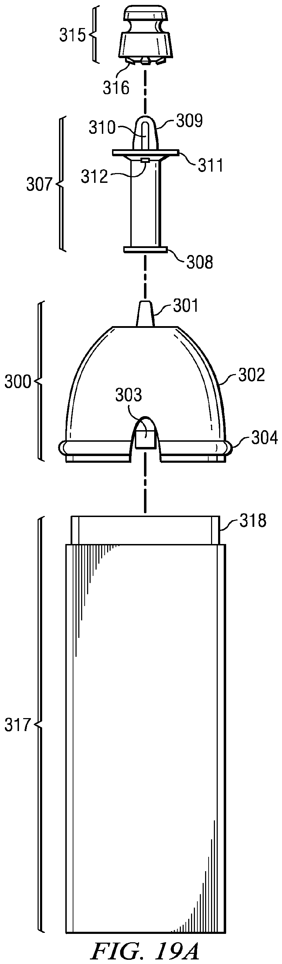

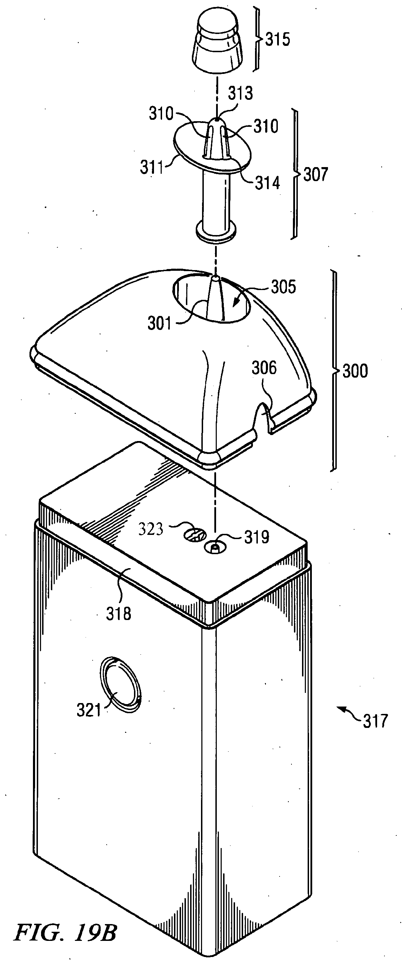

**Figures 19A** and **19** **B** show an exploded view of a portable nasal nebulizer in accordance with an embodiment of the present invention. The portable nebulizer comprises four sections. The first major section is the main canister **300,** which comprises a reservoir **305** for receiving fluid. The main canister **300** further comprises an air exit port **301.** As depicted in the figures, the air exit port **301** may extend above the top edge of the main canister **301.** However, in alternate embodiments (not shown), the air exit port **301** may be even with or recessed within the edge or portions of the edge of the main canister **300.** While the reservoir is depicted as substantially circular, it should be appreciated that the reservoir may comprise any shape. In one embodiment, the reservoir comprises an oval shape. Preferably, the reservoir should be shaped to allow for the receipt of a maximum amount of fluid.

Returning to the embodiment depicted beginning at **Figure 19A****,** the main canister further comprises a curved wall **302** surrounding the opening to the reservoir **305.** The curved wall **302** comprises a convex shape that extends downwardly around the periphery of the opening into a bottom generally rectangular opening configured to mate with a pressurized air supply, as further discussed below. When viewed from below, the main canister **300** thus comprises a generally hollow portion surrounding the reservoir portion **305.**

The second major section of the portable nebulizer is the insert **307,** which comprises a base **308** that fits within the reservoir section **305** of the canister. As depicted in **Figures 19A** and **19B****,** the base **308** is circular. However, the base may comprise any number of shapes so long as it fits within the canister. The insert comprises a fluid channel **309** with one end at the bottom of the reservoir **305** and one end that is positioned in the airstream so that the airstream creates a negative pressure in each tube that draws fluid into the airstream where it is atomized. The end positioned in the airstream comprises an exit hole **313.** The fluid channel **309** is slightly larger in diameter than the air exit port **301** of the main canister **300,** thereby providing a small space (preferably 0.0001" to 0.010" (0.00254 - 0.254 mm)) between the outer surface of the air exit ports and the inner surface of the fluid channels. This space allows fluid from the reservoir **305** to proceed upward between the air exit port **301** and the fluid channel **301** until being expelled by pressurized air. When the insert **307** is installed in the main canister **300,** the orifice **313** of the fluid channel **301** is positioned relative to the air exit **301** so as to create a venturi effect with the pressurized gas. Because the fluid exit in the insert **313** is larger than the air exits **301,** when air is forced through the air exits at an appropriate volume and speed, fluid in the reservoir **305** is drawn up into the space between the insert and air exit port. Thus, when this fluid meets the subsequent airstream it is atomized into particles conducive to deposition in the upper airway. The airstream is sufficient to penetrate the nasal cavity above the inferior turbinate so as to deposit the fluid and provide a washing, irrigation, or deposition to the upper reaches the nasal cavity. The fluid channel size may be adjusted to change the particle size of the mist.

The insert **307** may be keyed in at least one location with the reservoir **305** to ensure that the insert does not rotate in relation to the exit port **301** of the main canister **300** and to aid in centering of the insert **307** and its fluid channel **309** on the air exit port **301.** In one embodiment, the insert may also include a feature to ensure that it is inserted into the main canister in only one orientation.

At least one channel is located in the bottom of the insert **307** to act as a conduit for fluid from the reservoir **305** to enter the base **308** of the insert. As best depicted above in **Figures 9b** **and** **12b****,** the bottom face of the base **308** of the insert **307** comprises at least one channel or groove that forms a communication channel between the canister and the insert. The groove extends from the outside of the base to the inside of the insert. The base should comprise at least one groove but may also comprise more than one, as depicted in **Figure 9b****.** The number of grooves as well as the width and depth of the groove will help regulate the flow of fluid up to the point that the airflow takes over the upper limit of flow. In one embodiment, the grooves may range in width from about 0.005" to about 0.150" (0.127 mm to about 3.81 mm). In one embodiment, the grooves may range in depth from about 0.001" to about 0.050" (0.0254 to about 1.27 mm).

The canister **300** and the insert **307** may or may not be affixed together to form one integral piece. The bond may be formed by any means known in the art including without limitation use of a solvent bond, glue UV-cured adhesives, mechanical attachment, heat forming, or radiofrequency or ultrasonic welding. Alternatively, the canister and insert may be affixed together via a mechanical interlocking element such as a friction fit or a snap fit to form a temporary connection.

The insert further comprises an extension **311.** As depicted in **Figures 19A** **and** **19B****,** the extension **311** protrudes outwardly from the insert **307.** The extension **311** may extend from any point along the insert to form a top, or lid, to the canister **300.** In one embodiment, the extension substantially covers the opening of the reservoir **305.** In another embodiment, the extension entirely covers the opening of the reservoir **305.** In one embodiment, the extension comprises a downward concave shape relative to a plane substantially perpendicular to the fluid channel; or, relative to the top surface of the lid. In one embodiment, the extension comprises a two-step diameter (not shown) to mate with a rim of the opening. The insert **307** further comprises one or more apertures **314** around the fluid channel, each of the apertures lining up with a vertical groove **310** along the exterior of the fluid channel **309.** The groove **310** runs vertically from a point below the exit hole of the fluid channel **309** down to an aperture **314** in the extension **311.** During use, the deflected fluid will begin to flow back down the vertical groove **310.** The aperture **314** communicates with the inner chamber formed between the main canister **300** and insert **307.** As fluid exits the inner reservoir, a vacuum is created that actually pulls the deflected fluid back into the reservoir **305** through the aperture **314,** thereby ensuring maximum usage and minimized waste of the fluid.

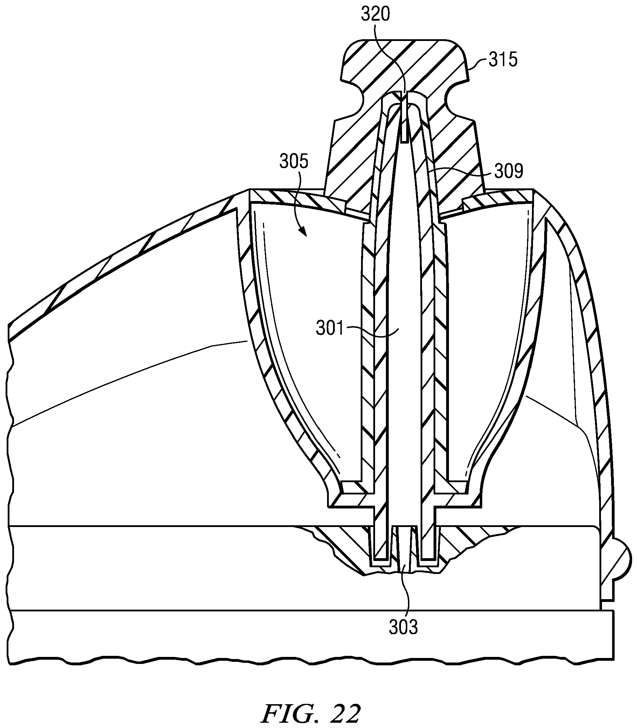

Another section of the portable nasal nebulizer is a removable cap **315** of the nasal nebulizer. The cap **315** comprises no holes and fits over and substantially covers the fluid channel **309.** Optionally, the cap may comprise a flattened edge (as shown above in **Figure 12A****)** to help with alignment with the apertures **314** of the insert **307** and also help with the grasping the cap **315.** The bottom portion of the cap should mate with a portion of the top face of the extension. The cap **315** further comprises one or more projections **312** on its bottom face, which mates with the apertures **314** of the extension. The number of projections **312** on the bottom face of the cap **315** should equal the number of apertures **314** in the insert **307.** As best depicted in **Figure 22****,** the cap comprises a projection or sealing plug **320** that projects into and fits within the exit hole **313** of the fluid channel and extending into the air exit port **301** of the canister to seal the reservoir from the air exit port and fluid channel exit when the cap is placed over the insert.

A fourth section of the portable nasal nebulizer is a handheld pressurized air supply source **317** onto which the main canister **300** fits. Preferably, the pressurized air supply source is a handheld air compressor. As shown in **Figure 19B****,** the air supply source comprises an air outlet **319,** which connects with the air inlet **303** of the main canister. In one embodiment, the canister snap fits onto the pressurized air supply source **317** to form an airtight seal between the air inlet **303** and the air outlet **319.** In one embodiment, the airtight seal may comprise an O-ring or soft plastic portion between the air inlet **303** and the air outlet **319** (not shown). An air input **323** supplies air to the pressurized air supply source **317** and may comprise a filter to keep out foreign materials. In order to accommodate for the air input **323,** the main canister **300** comprises an air vent **306,** which allows air into the air input **323** without interrupting the airtight seal between the canister **300** and air supply source **317.** The bottom rim **304** surrounding the generally rectangular bottom of the main canister **300** is fashioned to fit onto the pressured air supply source **317** such that no wiring or connecting tubing is required. Thus, unlike previous embodiments, a foot section at the bottom of the main canister is not necessary in order to stabilize the canister on a substantially flat surface. Instead, the pressurized air supply connects directly and immediately with the main canister.

While the pressurized air supply source **317** is depicted as having a generally rectangular shape, the source **317** may comprise any shape so long as it remains portable and capable of directly attaching to the main canister without the use of tubing. In one embodiment, the pressurized air supply source **317** is substantially rectangular. Preferably, the pressurized air supply source comprises an ergonomic shape to increase user comfort. For example, the air supply source **317** may comprise a grasping or gripping portion having a shape that corresponds to a palm of a hand of the user. The gripping portion may be on one side of the air supply source, with a second opposing side substantially flat; or it may comprise curves substantially around the entire periphery of the air supply source such that user may hold the portable device lengthwise with his or her hand around substantially the entire pressurized air supply source **317.** In one embodiment, the air supply source **317** comprises an ergonomic grasping portion. In another embodiment, the pressurized air supply source **317** is substantially rectangular with curves and features that make it easy to hold in the hand. In order to allow for portability of the nebulizer device, the pressurized air supply should generally be small enough to easily carry or transport. In one embodiment, the pressurized air supply source comprises a ratio of width: length: depth of about 2.5:3:1. In another embodiment, the pressurized air supply source comprises a ratio of width: length: depth of about 9:15:5. In one embodiment, the pressurized air supply source comprises a ratio of width: length: depth of between about 2.5:3:1 to about 9:15:5. By way of example, in one embodiment, the length may be about 15.5 cm, the width may be about 9.2 cm, and the depth may be about 5.7 cm. It should be recognized that any number of sizes and dimensions is possible while maintaining portability.

The pressurized air supply source **317** may employ an AC/DC power supply. The source **317** is DC-operated and may include a rechargeable internal battery or an external, detachable battery for easy exchange of depleted batteries. The source **317** may further be operated using a power switch **321** capable of turning on the air supply. The switch **321** may be an intermittent switch conveniently located on the air supply source **317** such that a user may conveniently reach it with one of his or her fingers. In one embodiment, the air supply source **317** may also comprise an indicator for the level of charge on the battery (not depicted) or a timer that beeps at timed intervals to deliver medication evenly between nostrils (not depicted). As described above, the pressurized air has a pressure of 0.069 - 1.035 bar and an airflow rate of 1 - 12 liters per minute, producing a fluid delivery rate of 1 - 20 ml per minute.

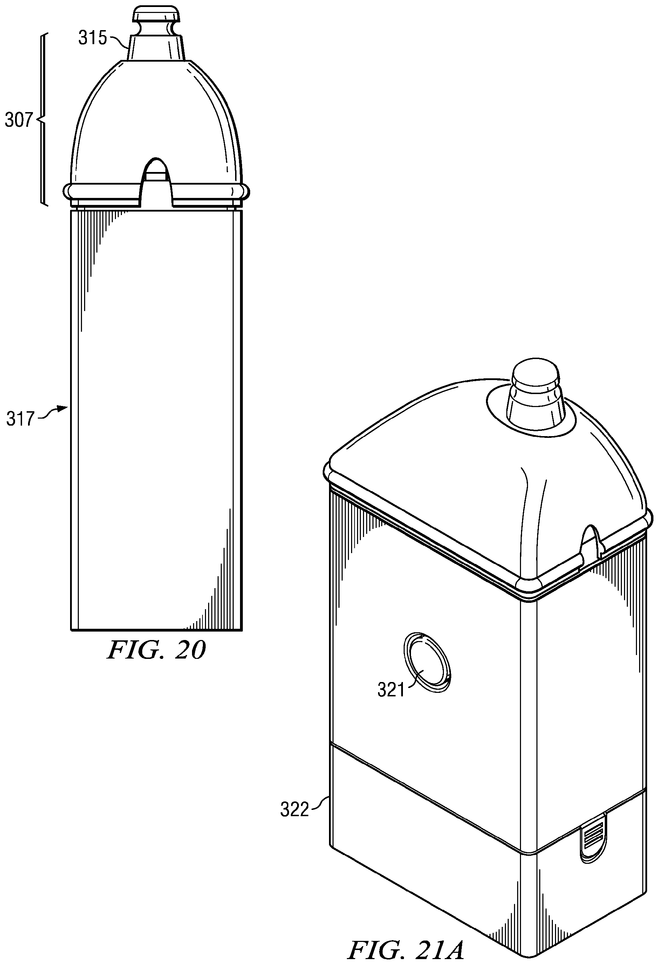

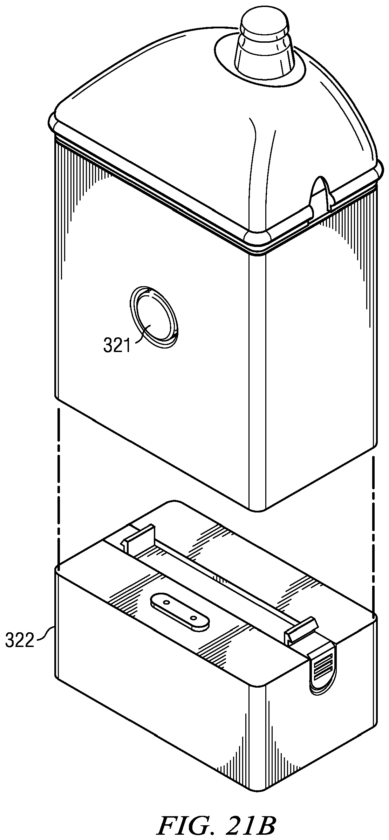

**Figure 20** shows a front perspective view of an assembled portable nebulizer as shown in **Figures 19A** and **19B****,** with the removable cap positioned over the device. Thus, when fully assembled with the cap in place, the portable nebulizer device is completely self-contained, prohibiting any leakage of fluids. As depicted in **Figures 19A** and **19B****,** in one embodiment, the pressurized air supply source **317** comprises an internal battery, which may or may not be rechargeable. **Figure 21A** shows a perspective view of an assembled portable nebulizer in another embodiment, with a detachable battery compartment **322** for one or more batteries which may or may not be rechargeable. In this embodiment, the battery compartment may detach from a portion of the pressurized air device by way of a switch element. **Figure 21B** shows a perspective view of a portable nebulizer as depicted in **Figure 21A****,** with the battery compartment **322** detached from the air supply source **317.**

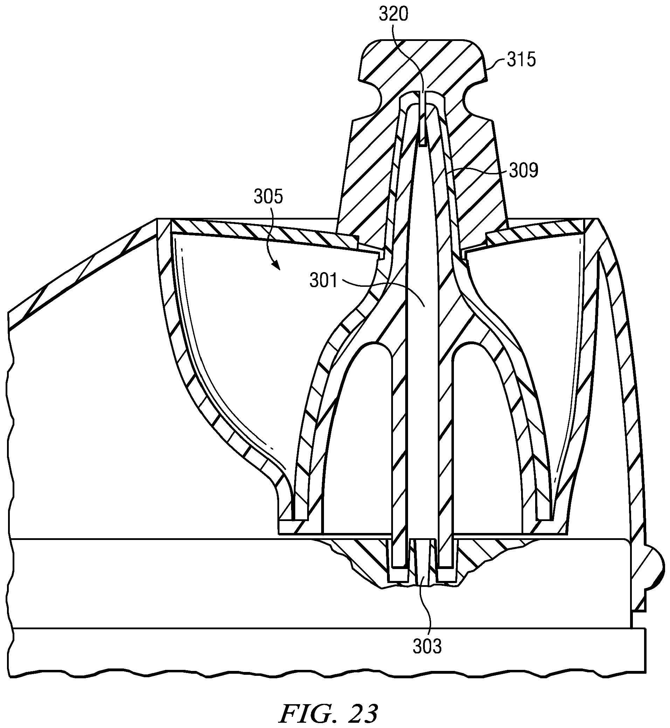

**Figure 22** shows a cross sectional detailed view of the main canister **300,** insert **307** and cap **315** portions in an assembled portable nebulizer according to one embodiment of the present disclosure. As best depicted here, the air exit port **301** and fluid channel **309** form two overlapping, concentric, tapered tubed having the requisite gap or space, as described above, between them in order to allow for the venturi effect. When connected to the pressurized air supply source **317,** the air inlet of the main canister plugs directly the supply source or air compressor by way of its air outlet. An alternate embodiment depicted in **Figure 23** shows that the air exit port **301** and the fluid channel **309** may also include a common bell housing as with previous embodiments.

By way of example, a portable nasal nebulizer device as described herein may be comprised of ABS, Polycarbonate, glass, stainless steel, styrolene, styrene-butadiene copolymer, or any other plastics appropriate for medical device use, and any combination thereof. The device may further be comprised of an antimicrobial compound in some embodiments.

The invention illustratively disclosed herein suitably may be practiced in the absence of any element, which is not specifically disclosed herein. It should also be notes that the invention is not limited to human use, but may also be used with any number of mammals including without limitation equine, canine, feline, non-human primate, rodent, bovine, and porcine.

The description of the present invention has been presented for purposes of illustration and description, and is not intended to be exhaustive or limited to the invention in the form disclosed. Many modifications and variations will be apparent to those of ordinary skill in the art. The embodiment was chosen and described in order to best explain the principles of the invention, the practical application, and to enable others of ordinary skill in the art to understand the invention for various embodiments with various modifications as are suited to the particular use contemplated. It will be understood by one of ordinary skill in the art that numerous variations will be possible to the disclosed embodiments without going outside the scope of the invention as disclosed in the claims.

## Claims

1. A portable nasal nebulizer, comprising:
(a) a main canister (300) having a rim, the canister comprising at least one air exit port (301); and
(b) an insert (307) with a base that fits within the canister, wherein the insert (307) comprises at least one fluid channel that fits over the air exit port, and further wherein the fluid channel is larger in diameter than the air exit port, thereby providing a small space between the outer surface of the air exit port and the inner surface of the fluid channel; and
(c) a pressurized air supply source (317) for introducing pressurized air through the air exit port such that the fluid is expelled as a mist through an exit hole in the fluid channel, wherein the insert comprises an extension protruding outwardly to the canister, **characterized in that**:
the air exit port (301) extends beyond the rim of the canister; the pressurized air supply source (317) is contiguously attached without tubing to the canister; and the fluid channel further comprises a groove extending vertically along the exterior of the fluid channel to an aperture in the extension, said aperture creating a channel to the canister (317).

2. The portable nasal nebulizer of claim 1 wherein said main canister fits onto the pressurized air supply source.

3. The portable nasal nebulizer of claim 1 wherein said pressurized air supply source attaches directly to an air inlet of the main canister.

4. The portable nasal nebulizer of claim 1 wherein said pressurized air supply source comprises an air outlet that connects directly to an air inlet of the main canister.

5. The portable nasal nebulizer of claim 1 wherein said base comprises at least one groove along its bottom face forming a communication channel between the canister and the fluid channel.

6. The portable nasal nebulizer of claim 1 comprising a cap without holes therethrough that substantially covers the fluid channel of the insert, said cap plugging the exit hole of the fluid channel and the exit port.

7. The portable nasal nebulizer of claim 1 wherein said pressurized air supply source is an air compressor.

8. The portable nasal nebulizer of claim 1 pressurized air supply source comprises a battery source.

9. The portable nasal nebulizer of claim 8 wherein said battery source is internal.

10. The portable nasal nebulizer of claim 1 wherein said extension forms a lid over a portion of the canister.

11. The portable nasal nebulizer of claim 10 comprising a cap without holes therethrough, said cap comprising a sealing plug for sealing the air exit port and the fluid channel of the insert and further wherein said cap comprises a projection that fits within the aperture of the extension, thereby sealing the aperture.

12. The portable nasal nebulizer of claim 10 wherein the lid is downwardly concave.

13. The portable nasal nebulizer of claim 1 wherein said pressurized air supply source comprises a ratio of width: length: depth of about 2.5:3:1.

14. The portable nasal nebulizer of claim 1 wherein said pressurized air supply source comprises an ergonomic shape to increase user comfort.

## Patentansprüche

1. Tragbarer Nebulisator für die Nase, umfassend:
(a) ein Hauptbehältnis (300), das einen Rand aufweist, wobei das Behältnis mindestens eine Luftaustrittsöffnung (301) umfasst;
und
(b) einen Einsatz (307) mit einer Basis, der in das Behältnis passt, worin der Einsatz (307) mindestens einen Fluidkanal umfasst, der über die Luftaustrittsöffnung passt, und ferner worin der Fluidkanal im Durchmesser größer als die Luftaustrittsöffnung ist, wodurch ein kleiner Zwischenraum zwischen der äußeren Oberfläche der Luftaustrittsöffnung und der inneren Oberfläche des Fluidkanals vorgesehen ist; und
(c) eine Druckluftzufuhrquelle (317) für das Einbringen von Druckluft durch die Luftaustrittsöffnung in einer solchen Art und Weise, dass das Fluid als ein Sprühnebel durch ein Austrittsloch im Fluidkanal ausgestoßen wird, worin der Einsatz eine vom Behältnis nach außen vorstehende Erweiterung umfasst, **dadurch gekennzeichnet, dass**:
die Luftaustrittsöffnung (301) über den Rand des Behältnisses hinausragt; wobei die Druckluftzufuhrquelle (317) unmittelbar am Behältnis, ohne Verrohrung, angebracht ist; und der Fluidkanal ferner eine Nut umfasst, die sich vertikal entlang der Außenseite des Fluidkanals zu einer Öffnung in der Erweiterung erstreckt, wobei die Öffnung einen Kanal zum Behältnis (317) herstellt.

2. Tragbarer Nebulisator für die Nase nach Anspruch 1, worin das Hauptbehältnis auf die Druckluftzufuhrquelle passt.

3. Tragbarer Nebulisator für die Nase nach Anspruch 1, worin die Druckluftzufuhrquelle direkt an einem Lufteinlass des Hauptbehältnisses angebracht ist.

4. Tragbarer Nebulisator für Nase nach Anspruch 1, worin die Druckluftzufuhrquelle einen Luftauslass umfasst, der direkt mit einem Lufteinlass des Hauptbehältnisses verbunden ist.

5. Tragbarer Nebulisator für die Nase nach Anspruch 1, worin die Basis mindestens eine Nut entlang der Bodenfläche derselben umfasst, die einen Kommunikationskanal zwischen dem Behältnis und dem Fluidkanal bildet.

6. Tragbarer Nebulisator für die Nase nach Anspruch 1, umfassend eine ohne Löcher versehene Kappe, die den Fluidkanal des Einsatzes im Wesentlichen abdeckt, wobei die Kappe das Austrittsloch des Fluidkanals und die Austrittsöffnung verschließt.

7. Tragbarer Nebulisator für die Nase nach Anspruch 1, worin die Druckluftzufuhrquelle ein Luftkompressor ist.

8. Tragbarer Nebulisator für die Nase nach Anspruch 1, worin die Druckluftzufuhrquelle eine Batteriequelle umfasst.

9. Tragbarer Nebulisator für die Nase nach Anspruch 8, worin die Batteriequelle im Inneren ist.

10. Tragbarer Nebulisator für die Nase nach Anspruch 1, worin die Erweiterung einen Deckel über einen Abschnitt des Behältnisses bildet.

11. Tragbarer Nebulisator für die Nase nach Anspruch 10, der eine ohne Löcher versehene Kappe umfasst, wobei die Kappe einen Abdichtstopfen umfasst, um die Luftaustrittsöffnung und den Fluidkanal des Einsatzes abzudichten, und ferner worin die Kappe einen Vorsprung umfasst, der in die Apertur der Erweiterung hineinpasst, wodurch die Apertur abgedichtet wird.

12. Tragbarer Nebulisator für die Nase nach Anspruch 10, worin der Deckel nach unten konkav ist.

13. Tragbarer Nebulisator für die Nase nach Anspruch 1, worin die Druckluftzufuhrquelle ein Verhältnis von Breite : Länge : Tiefe von ungefähr 2,5 : 3 : 1 umfasst.

14. Tragbarer Nebulisator für die Nase nach Anspruch 1, worin die Druckluftzufuhrquelle eine ergonomische Form zur Erhöhung des Benutzerkomforts umfasst.

## Revendications

1. Nébuliseur nasal portatif, comprenant :
(a) une cartouche principale (300) ayant un rebord, la cartouche comprenant au moins un orifice de sortie d'air (301) ; et
(b) un insert (307) ayant une base qui s'ajuste dans la cartouche, dans lequel l'insert (307) comprend au moins un canal de fluide qui s'ajuste sur l'orifice de sortie d'air, et dans lequel en outre le canal de fluide a un diamètre plus grand que l'orifice de sortie d'air, fournissant ainsi un petit espace entre la surface extérieure de l'orifice de sortie d'air et la surface intérieure du canal de fluide ; et
(c) une source d'alimentation en air sous pression (317) pour introduire de l'air sous pression à travers l'orifice de sortie d'air de sorte que le fluide est expulsé sous forme de brume à travers un trou de sortie dans le canal de fluide, dans lequel l'insert comprend un prolongement faisant saillie vers l'extérieur de la cartouche, **caractérisé en ce que** :
l'orifice de sortie d'air (301) s'étend au-delà du rebord de la cartouche ; la source d'alimentation en air sous pression (317) est attachée de manière contiguë sans tubulure à la cartouche ; et le canal de fluide comprend en outre une rainure s'étendant verticalement le long de l'extérieur du canal de fluide jusqu'à une ouverture dans le prolongement, ladite ouverture créant un canal allant jusqu'à la cartouche (317).

2. Nébuliseur nasal portatif selon la revendication 1, dans lequel ladite cartouche principale s'ajuste sur la source d'alimentation en air sous pression.

3. Nébuliseur nasal portatif selon la revendication 1, dans lequel ladite source d'alimentation en air sous pression s'attache directement à une entrée d'air de la cartouche principale.

4. Nébuliseur nasal portatif selon la revendication 1, dans lequel ladite source d'alimentation en air sous pression comprend une sortie d'air qui se connecte directement à une entrée d'air de la cartouche principale.

5. Nébuliseur nasal portatif selon la revendication 1, dans lequel ladite base comprend au moins une rainure le long de sa face inférieure formant un canal de communication entre la cartouche et le canal de fluide.

6. Nébuliseur nasal portatif selon la revendication 1 comprenant un capuchon sans trous à travers celui-ci qui recouvre sensiblement le canal de fluide de l'insert, ledit capuchon s'enfichant dans le trou de sortie du canal de fluide et l'orifice de sortie.

7. Nébuliseur nasal portatif selon la revendication 1, dans lequel ladite source d'alimentation en air sous pression est un compresseur d'air.

8. Nébuliseur nasal portatif selon la revendication 1, dans lequel la source d'alimentation en air sous pression comprend une source de batterie.

9. Nébuliseur nasal portatif selon la revendication 8, dans lequel ladite source de batterie est interne.

10. Nébuliseur nasal portatif selon la revendication 1, dans lequel ledit prolongement forme un couvercle sur une partie de la cartouche.

11. Nébuliseur nasal portatif selon la revendication 10 comprenant un capuchon sans trous à travers celui-ci, ledit capuchon comprenant un bouchon d'étanchéité pour sceller l'orifice de sortie d'air et le canal de fluide de l'insert et dans lequel en outre ledit capuchon comprend une saillie qui s'ajuste dans l'ouverture du prolongement, scellant ainsi l'ouverture.

12. Nébuliseur nasal portatif selon la revendication 10, dans lequel le couvercle est concave vers le bas.

13. Nébuliseur nasal portatif selon la revendication 1, dans lequel ladite source d'alimentation en air sous pression affiche un rapport largeur:longueur:profondeur d'environ 2,5:3:1.

14. Nébuliseur nasal portatif selon la revendication 1, dans lequel ladite source d'alimentation en air sous pression a une forme ergonomique pour augmenter le confort de l'utilisateur.
